Europäisches Patentamt

European Patent Office

Office européen des brevets

(19)

(11) Publication number: **0 171 128**
A1

(12)

# EUROPEAN PATENT APPLICATION

(21) Application number: **85300902.5**

(22) Date of filing: **11.02.85**

(51) Int. Cl.⁴: **C 12 N 15/00,** C 12 N 1/20, C 12 P 19/34 // C12R1:19

(30) Priority: **08.08.84 US 638975**

(43) Date of publication of application: **12.02.86** **Bulletin 86/7**

(84) Designated Contracting States: **AT BE CH DE FR GB IT LI LU NL SE**

(71) Applicant: **QUIDEL, 11077 North Torrey Pines Road, La Jolla, CA 92037 (US)**

(72) Inventor: **Marquis, David M., 11331 Pegasus Avenue, San Diego California 92126 (US)**
Inventor: **Smolec, Jo Marie, 11473 Madera Rosa Way, San Diego California (US)**

(74) Representative: **Sommerville, John Henry et al, SOMMERVILLE & RUSHTON 11 Holywell Hill, St. Albans Hertfordshire, AL1 1EZ (GB)**

(54) **T-cell growth factor.**

(57)  cDNA, which codes for TCGF, plasmids containing the same, and E. coli which contain such plasmids and express such cDNA suitable for the production of TCGF are disclosed.

EP 0 171 128 A1

- 1 -

## T-CELL GROWTH FACTOR

### TECHNICAL FIELD

The invention relates to the cloning of a complementary deoxyribonucleic acid (cDNA) sequence coding for a T cell growth factor (TCGF).

### BACKGROUND OF THE INVENTION

#### The Immune Response

The immune response to a given antigen is in most cases supported by multiple cell types of the reticulo-endothelial system. For example, humoral immunity is mediated by antibodies produced by B lymphocytes (B cells), whereas cellular immunity is mediated by specially sensitized T lymphocytes (T cells). The interaction of these cells and others is further modulated by various mediators. T cells that have been stimulated by antigens or non-specific mitogens have been shown to produce antigen-specific mediators, or lymphokines, that regulate humoral and cell-mediated immune responses in both a positive and a negative manner, as well as nonantigen-specific mediators that are associated with delayed hypersensitivity and B and T cell collaboration to produce antibodies. The biological significance and the structure-function relationship of these various lymphokines have proven difficult to determine because a simple stimulation produces a mixture of lymphokines in extremely low concentrations, making purification and subsequent study and assignment of a particular biological activity to a given lymphokine difficult.

- 2 -

Efforts to develop a more homogeneous and abundant source of lymphokines have resulted in the establishment of homogeneous T cell lines secreting a distinct or a limited number of lymphokines. Although neoplastic lines are capable of long-term growth, immortalization of normal T cells has proved unsuccessful unless repeatedly stimulated with an antigen, until it was discovered that medium that had been conditioned by mitogen stimulated T cells supported long-term growth of the normal T cell lines. (Morgan, D.A., et al., "Selective in vitro growth of T lymphocytes from normal human bone marrows", Science 193: 1007, 1976).

The mediator responsible for stimulating T cell growth has been termed T cell growth factor (TCGF or interleukin-2) and its discovery marked an important advance in the understanding of the mechanisms of proliferation of T cells. (Altman, A. and D.H. Katz, "The biology of monoclonal lymphokine secreted by T cell lines and hybridomas", Adv. Immunol. 33: 73, 1982, herein referred to as Altman and Katz, 1982). The mechanism involves the concerted interaction of several discrete subsets of T cells which, through the mediation of antigens and other lymphokines, notably lymphocyte-activating factor (LAF or interleukin-1), establishes and maintains a reservoir of T cells which both respond to and synthesize TCGF, thereby serving as a growth and differentiation factor for T Cells.

In addition to the relevance of TCGF to normal immunological responses, recent studies reported a T cell line derived from a malignant lymphoma becoming independent of exogenous TCGF after several weeks of culture by secreting their own TCGF, implicating a potential role of TCGF in the maintenance of T cell malignancies. (Gootenberg, J.E., et al., "Human cutaneous T cell lymphoma and leukemia cell lines produce and respond to T cell growth factor", J. Exp. Med. 154: 1403, 1981). Investigators have also shown that natural killer cell activity may be stimulated by TCGF, indicating its potential use in the treatment of malignancies. (Henney, C.S., et al., "Interleukin-2 augments natural killer cell activity", Nature 291: 335, 1981). More recently, preliminary investigations have indicated that AIDS (Acquired Immune Deficiency Syndrome) is characterized by a severe deficiency of T cells, which may be amenable to TCGF treatment. Further, previous studies have yielded conflicting interpretations concerning the availablity of TCGF to act directly on B cells, namely by replacing the need for T cell help in antibody responses to T-dependent antigens in vitro (Altman and Katz, 1982).

However, the most convincing data on this point comes from studies by Harwell, et al. "Concanavalin A-inducible, interleukin-2-producing T cell hybridoma", J. Exp. Med. 152: 893, 1980, who demonstrated that TCGF produced by a Con A-inducible T cell hybridoma lacked the capacity to

- 4 -

trigger B cells directly when stringent methods to eliminate all T cells from the responding population were employed.

Conventional preparations of TCGF contain a protein with a molecular weight of 14,000-16,000. Although extensive purification has been recently achieved, particularly of murine and human TCGF (see, for example, Mier, J.W. and R.C. Gallo, "The purification and properties of human T cell growth factor", J. Immunol. 128: 1122, 1982), the very low yield of purified material has often precluded further characterization.

It is clear that TCGF exists physiologically only in very small amounts, and even the use of the more advanced cell cloning techniques have still only produced minute quantities of biologically active materials. Although human TCGF stimulates the proliferation of non-human cell lines, non-human TCGF apparently is not active on human T cells, thereby eliminating other animals as a source of TCGF for human use.

Perhaps in no research area other than lymphokine or cytokine biology is the application of recombinant DNA technology more urgently required to enable definitive progress to be made towards; (1) delineating the precise biological activity inherent in TCGF; (2) elucidating the clinical usefulness of TCGF; and (3) establishing effective strategies for reproducible expression of the relevant gene product in unrestricted and economically

- 5 -

feasible quantities for use in the appropriate clinical arena.

### Recombinant DNA Technology

The use of cultured microorganisms such as yeast and bacteria to produce specific products is ancient. However, until the advent of recombinant DNA technology, the cellular products were essentially natural to the particular cell species producing them. The result of technological advances in recombinant DNA (genetic engineering ) now make possible the production of a protein by a cell previously having no genetic information coding for that protein. The immediate advantage of this is to have a protein normally produced by a cultured cell line that is difficult and expensive to maintain, such as most mammalian cell lines, now being produced by a cell line such as E.coli, whose growth parameters are well known and is inexpensive to cultivate in large quantities.

The basis of recombinant DNA technology resulting in the production of a foreign protein by a cell is the ability to isolate or produce the gene(s) or gene fragments coding for the protein and transfer this genetic information to a host microorganism; this transferred genetic material is then replicated as the microorganism proliferates. Cell progeny are capable of producing the protein which can then be isolated in a much greater quantity than heretofore possible. A more detailed explanation of the biological facts underlying the basic methodology and its application has been reported by Ullrich, A., et al. "Rat insulin genes: Construction of plasmids containing the coding sequences", Science 196: 1313, 1977, as well as by Baxter, J.D., et al. (U.S. Pat. No. 4,322,499), which are incorporated herein by reference.

The desired gene or gene fragment coding for a particular protein is either isolated or synthesized in vitro using the messenger RNA (mRNA), known to code for the production of the protein, as a template, as described herein. In order that the newly synthesized DNA, termed complementary DNA (cDNA), may be transferred to the host cell in a form capable of replication, it is first incorporated into a transfer vector. The transfer vector is a DNA molecule that contains sufficient genetic information to enable it to replicate once it is transferred to the host cell. Two commonly used transfer vectors are plasmids and phages. Plasmids are generally double-stranded DNA ring structures of a microbial cell, separate from the chromosomal DNA, and easily isolated from the chromosomal DNA by virtue of their much smaller size. Generally a plasmid used as a transfer vector will contain a particular gene as a marker, for example, ampicillin resistance, making isolation of the plasmid-containing host cells less difficult.

The new cDNA gene(s) is incorporated into the plasmid by first cleaving the plasmid with an enzyme that is site- specific (restriction endonuclease). A variety of restriction endonucleases have been isolated and characterized in terms of the nucleotide sequence of their recognition sites. Since the new cDNA requires a proximally-located control region in order that expression may occur (via transcription and translation), particular endonucleases are used to achieve this goal.

The transfer of the new plasmid vector to the host cell (transformation) is not clearly understood, but can be maximized by certain treatments. Once incorporated into the cell, the plasmid is capable of replicating independently of cellular chromosomal replication and is transferred to the cell progeny upon duplication. Although plasmid vectors have been used as the transfer vector for the invention described herein, it will be understood that other types of transfer vectors may be employed.

In summary, with the use of recombinant DNA technology, a microorganism can be made capable of producing a mammalian protein by, (1) synthesizing the appropriate DNA coding for the protein, (2) incorporating the gene or genetic material into a self-replicating vector, and (3) inserting the plasmid vector into a host microorganism. Under appropriate environmental conditions, plasmid replication and cell duplication result in the synthesis of the desired protein.

- 8 -

## SUMMARY OF THE INVENTION

In one form, the present invention comprises the bacterium Escherichia coli C600/QUIDL-TF, as described as to method of preparation and, specifically, as to cDNA sequence hereinafter in complete detail.

In another aspect, the invention comprises a plasmid containing a substantial portion of the following sequence of DNA which codes for for T-Cell Growth Factor:

GGGGGGGGGGGGGGGGGTACAGGATGCAACTCCTGTCTTGCATTGCACTAA

GTCTTGCACTTGTCACAAACAGTGCACCTACTTCAAGTTCTACAAAGAAA

ACACAGCTACAACTGGAGCATTTACTGCTGGATTTACAGATGATTTTGAA

TGGAATTAATAATTACAAGAATCCCAAACTCACCAGGATGCTCACATTTA

AGCTTTACATGCCCAAGAAGGCCACAGAACTGAAACATCTTCAGTGTCTA

GAAGAAGAACTCAAACCTCTGGAGGAAGTGCTAAATTTAGCTCAAAGCAA

AAACTTTCACTTAAGACCCAGGGACTTAATCAGCAATATCAACGTAATAG

TTCTGGAACTAAAGGGATCTGAAACAACATTCATGTGTGAATATGCTGAT

GAGACAGCAACCATTGTAGAATTTCTGAACAGATGGATTACCTTTTGTCA

AAGCATCATCTCAACACTGACTTGATAATTAAGTGCTTCCCACTTAAAAC

ATATCAGGCCTTCTATTTATTTAAATATTTAAATTTTATATTTATTGTTG

AATGTATGGTTTGCTACCTATTGTAACTATTATTCTTAATCTTAAAACTA

TAAATATGGATCTTTTATGATTCTTTTTGTAAGCCCTAGGGGCTCTAAAA

TGGTTTCACTTATTTATCCCAAAATATTTATTATTATGTTGAATGTTAAA

TATAGTATCTATGTAGATTGGTTAGTAAAACTATTTAATAAATTTGATAA

ATATAAAAAAACACCCCCCCCCCCCCCCCCCCCCCCCCCCC

The invention comprises, in one facet, a strain of the bacteria <u>Escherichia</u> <u>coli</u> containing a plasmid which includes a substantial portion of the aforesaid DNA sequence which codes for the expression of T-Cell Growth Factor therein.

The present invention includes a technique suitable for cloning cDNA having a coding sequence capable of expressing the polypeptide TCGF.

The invention also consists essentially of the DNA sequence.

GGGGGGGGGGGGGGGGGTACACGATGCAACTCCTGTCTTGCATTGCACTAA

GTCTTGCACTTGTCACAAACAGTGCACCTACTTCAAGTTCTACAAAGAAA

ACACAGCTACAACTGGAGCATTTACTGCTGGATTTACAGATGATTTTGAA

TGGAATTAATAATTACAAGAATCCCAAACTCACCAGGATGCTCACATTTA

AGCTTTACATGCCCAAGAAGGCCACAGAACTGAAACATCTTCAGTGTCTA

GAAGAAGAACTCAAACCTCTGGAGGAAGTGCTAAATTTAGCTCAAAGCAA

AAACTTTCACTTAAGACCCAGGGACTTAATCAGCAATATCAACGTAATAG

TTCTGGAACTAAAGGGATCTGAAACAACATTCATGTGTGAATATGCTGAT

GAGACAGCAACCATTGTAGAATTTCTGAACAGATGGATTACCTTTTGTCA

AAGCATCATCTCAACACTGACTTGATAATTAAGTGCTTCCCACTTAAAAC

ATATCAGGCCTTCTATTTATTTAAATATTTAAATTTTATATTTATTGTTG

AATGTATGGTTTGCTACCTATTGTAACTATTATTCTTAATCTTAAAACTA

TAAATATGGATCTTTTATGATTCTTTTTGTAAGCCCTAGGGGCTCTAAAA

TGGTTTCACTTATTTATCCCAAAATATTTATTATTATGTTGAATGTTAAA

TATAGTATCTATGTAGATTGGTTAGTAAAACTATTTAATAAATTTGATAA

ATATAAAAAAACACCCCCCCCCCCCCCCCCCCCCCCCCCCC

including the operative portions thereof comprising from approximately nucleotide No. 50 to 500, and plasmids which include the same, as well as E. coli containing such plasmids.

In a more specific manner, the present invention comprises the DNA nucleotide sequence which codes for TCGF including the sequence TTTAAGCTTTACAT, including nucleotide No. 203, as numbered above, and plasmids which include the same, as well as E. coli containing the same.

The invention comprises the recombinant DNA transfer vector containing DNA having a sufficient portion of the sequence shown above to code for the expression of TCGF in a host, and a microorganism host including the aforesaid

- 11 -

expressing the polypeptide TCGF.

Messenger RNA was produced by stimulating the human T cell leukemia line JURKAT C9II with phytohemagglutinin and purified by chromotography.

DNA complementary to the isolated mRNA was synthesized using AMV reverse transcriptase. The Klenow fragment of E. coli polymerase was used for the second strand synthesis. The TCGF gene cDNA was combined with the plasmid pBR322 at the Pst I site and transformed into E. coli C600.

Following normal cell growth and division, the replicated colonies containing the cDNA were screened using available hybridization techniques. The cloned cDNA was found to consist of approximately 790 base pairs and its DNA sequence showed it to contain the TCGF gene.

## DETAILED DESCRIPTION OF THE INVENTION

### Definitions

The following abbreviations are used in the specification which follows, along with other terms which are defined as used:

| | |
|---|---|
| RPMI 1640 medium | A standard nutrient medium consisting of salts, vitamins, sugars and amino acids, Rosewell Park Medical Institute |
| HEPES buffer | A standard buffer comprising N-2-hydroxy-pipirazine-N'-2'-ethaneamine sulfonic acid, Calbiochem |
| Triton X-100 | A standard wetting agent of the |

- 12 -

hydroxy-pipirazine-N'-2'-ethaneamine
sulfonic acid, Calbiochem

Triton X-100      A standard wetting agent of the
octyl-phenoxy polyethoxy ethanol
class, Sigma Chemical Co.

BSA      Bovine serum albumin

FCS      Fetal calf serum

EDTA      Ethylenediamine tetraacetate disodium
salt, Fisher

AMV      Avian myeloblastosis virus

dNTP      Deoxynucleotide triphosphate,
Boehringer Mannheim

SDS      Sodium dodecyl sulphate, Bethesda
Research Laboratories

PHA      Phytohemagglutinin

PMA      Phorbol myristic acetate

This invention involves the formation of a cDNA molecule containing a gene that codes for TCGF or a TCGF-like protein, and the combination of the cDNA and a suitable transfer vector capable of transforming a host organism. More particularly, this invention involves the formation of a recombinant plasmid molecule used to transform E. coli, and the subsequent replication of the recombinant plasmid.

The description of the invention is divided into the following stages:

     I.      Selection and preparation of a transfer vector

- 13 -

II.  Source and assay of TCGF and TCGF mRNA

III.  Synthesis of cDNA

IV.  Construction of chimera (pBR322 with cDNA insert)

V.  Identification and characterization of the cloned cDNA for TCGF

VI.  Applications of the invention

I.  <u>Selection and Preparation of a Transfer Vector</u>.

The present invention provides a means by which a mammalian protein may be produced in a microorganism <u>in vivo</u> via recombinant DNA technology.  <u>In vivo</u> synthesis may occur when the desired gene coding for the protein has been combined with a transfer vector that permits transformation of the host microorganism.  It is essential that the transfer vector is capable of (a) incorporation into the host, (b) self-replication coordinately with the replication of the chromosomal DNA of the host cell, (c) expression of the inserted gene via transcription and translation, and (d) non-degradation of the resultant protein product which is formed in substantial amounts.

A wide variety of vectors are available that meet the necessary criteria.  In the preferred embodiment of this invention, the plasmid pBR322 was selected as the transfer vector.  pBR322 has been fully characterized by a restriction endonuclease map, incorporated herein by reference, (Sutcliffe, J. G., "Complete nucleotide sequence of the <u>Escherichia</u> <u>coli</u> plasmid pBR322", <u>Cold</u>

- 14 -

Spring Harbor Symposium 43:77, 1979.), and is a replicating plasmid which exhibits both ampicillin and tetracycline resistance ($Amp^R$ and $Tet^R$, respectively).

II.   Source and Assay of TCGF and TCGF mRNA.

A clone of the JURKAT human T-cell leukemia line (C911), prepared by limiting dilution and selected in the applicants' laboratory for its elevated production of TCGF, was grown in spinner culture in RPMI 1640 medium (M.A. Bioproducts, Walkersville, Md.) supplemented with 100 units/ml penicillin-100 µg/ml streptomycin, 1 mM sodium pyruvate, 2mM L-glutamine, 10mM HEPES and 10 percent fetal calf serum (FCS; M.A. Bioproducts, Walkersville, Md.).

Three to six liters of C911 cells at a density of $1 - 1.5 \times 10^6$ cells/ml were stimulated to synthesize TCGF by the addition of 10 µg/ml of phytohemagglutinin (PHA; Gibco) and 10 ng/ml of phorbol myristic acetate (PMA; Sigma). Sixteen hours later the cells were harvested by centrifugation, washed twice with ice-cold 50 mM Tris/HCl buffer, pH 7.4, containing 100 mM NaCl and 1 mM EDTA (TNE) and suspended in TNE to 1/1,000 of the original culture volume.

The suspended C911 cells were spun down at 1200 rpm for 10 minutes at 4 degrees centigrade. The cells were washed once with 40 ml of RNase-free 0.9 percent sodium chloride solution, and resuspended in 7.5 ml of ice cold hypotonic solution (20 mM Tris/HCl pH 7.4, 10 mM NaCl, 3

mM Mg (CH$_3$CO$_2$) supplemented with 10 mM ribonucleoside-vanadyl complexes; Bethesda Research Laboratories, Inc.). The cell suspension was lysed by the addition of 2.5 ml of lysing buffer (hypotonic solution plus 5 percent sucrose, 1.2 percent Triton X-100). The cells were kept on ice for 30 minutes and then centrifuged at 3000 rpm for 5 minutes at 4 degrees centigrade. The supernatant fluid was poured into a tube containing 0.5 ml 2 M NaCl, 0.25 ml 0.25 M EDTA, and 0.5 ml 10 percent SDS and then extracted with phenol-chloroform (1:1) three times. The final aqueous phase was then extracted with chloroform-isoamyl alcohol (24:1). To the remaining aqueous phase was added 1/9 volume of 3 M sodium actate (pH 5.3) and 2 volumes of cold ethanol were used to precipitate the RNA at -100 degrees centigrade overnight. The RNA was recovered by centrifugation at 10,000 rpm for 30 minutes at 4 degrees centigrade. The polyadenylated RNA was prepared by a single passage over an oligo(dT) cellulose (Type III; Collaborative Research, Inc.) column, as described in Aviv, H., and P. Leder, "Purification of biologically active globin messenger RNA by chromotography on olio-thymidylic acid-cellulose", Proc. Natl. Acad. Sci. 69: 1408, 1972.

The assay for TCGF is premised on the total dependency of certain cell lines on TCGF for successful growth in culture. The proliferation of these cells is determined by the uptake of tritiated thymidine which

- 16 -

quantitates TCGF activity. (Gillis, S., et al., "T cell growth factor: parameters of production and a quantitative microassay for activity", J. Immunol. 120: 2027, 1978.) Murine (C57BL/6) tumor-specific cytotoxic T lymphocytes (CTLL-2) maintained in TCGF-supplemented RPMI-1640 medium containing 100 units/ml penicillin, 100 µg/ml streptomycin, 1 mM sodium pyruvate, 2mM L-glutamine, 10 mM HEPES, and 5 percent FCS were used for the assay. The cells were washed free of growth medium and resuspended in RPMI-1640 medium supplemented as above without TCGF. To serial dilutions of the TCGF sample to be assayed, (100 µl in 96-well microplates) (No. 3596 Costar, Inc., Cambridge, Ma.), cells ($1 \times 10^4$/well in 100 µl) were added and incubated at 37 degrees C in a humidified atmosphere of 5 percent $CO_2$ in air. Following 20 hours of culture, 1.0 µCi of $^3$H-thymidine was added to each microplate well and cultured for an additional 4 hours. Cultures were harvested onto glass fiber filter strips and $^3$H-thymidine incorporation determined by standard techniques.

III.  Synthesis of cDNA.

A variety of sources or methods exist for producing a DNA sequence or gene that contains the sequence coding for a particular protein. In the preferred embodiment of this invention, complementary DNA (cDNA) was prepared from mRNA from stimulated JURKAT C911 cells in accordance with conventional procedures, (Wickens, M.P. et al., "Synthesis of double-stranded DNA containing 50 mM Tris/HCl, pH 8.3 at 42 degrees C, 140 mM KCl, 10 mM $MgCl_2$, 30 mM 2-mercaptoethanol, 500 mM of each deoxynucleotide triphosphate, 20 µCi of $(\alpha-{}^{32}P)$-dCTP, 100 µg/ml of oligo $(dT)_{12-18}$ and 65 units of avian myeloblastosis virus (AMV) reverse transcriptase. After being assembled in an autoclaved 1.5 ml micro test tube on ice with a reaction volume of 50 µl, the tubes were mixed, and centrifuged briefly. Reactions were incubated at 42 degrees C for 1 hour. The reaction was stopped by chilling the tubes on ice. The mRNA first-strand hybrids were separated by placing the tubes in boiling water for 2 minutes, plunging immediately into ice water, and centrifuging for 30 seconds to pellet the denatured protein.

The second strand was synthesized by adding 45 µl of the first strand product, on ice, to 18.7 µl $H_2O$, 6.3 µl 1 M KCl, 18 µl 1 M K HEPES, pH 6.9, 18 µl 25 mM $MgCl_2$, 50 µl of 1 mM dNTP's, 40 µCi $(\alpha-{}^{32}P)$dCTP and 78 units of large fragment DNA polymerase (Bethesda Research Laboratories, Inc.).

The reaction was incubated for 3 hours at 15 degrees C, and stopped by chilling on ice and adding 1/10 volume of 0.25 M EDTA and 1/20 volume of 10 percent SDS. Each reaction was extracted at room temperature with 200 µl of phenol-chloroform-$H_2O$ (1:1:1). The aqueous phase was passed over a column of Sephadex G-50 pre-equilibrated and run in 10 mM Tris/HCl pH 8.0, 1 mM EDTA. The void volume

phenol-chloroform-$H_2O$ (1:1:1). The aqueous phase was passed over a column of Sephadex G-50 pre-equilibrated and run in 10 mM Tris/HCl pH 8.0, 1 mM EDTA. The void volume material was collected and precipitated with alcohol after the addition of NaCl to 0.3M. The DNA was recovered by centrifugation and dried.

Nuclease S1 was added to a final concentration of 15 units per 500 ng of double stranded CDNA. The digestion was performed at 37 degrees C for 15 minutes in accordance with the manufacturer's recommendations (Bethesda Research Laboratories, Inc.). This reaction was extracted at room temperature with an equal volume of phenol-chloroform-$H_2O$ (1:1:1)) and purified on a Sepharose 4B column using 10 mM Tris/HCl pH 8.0, 5mM NaCl, 1 mM EDTA, and 0.2 percent SDS as eluate. A small portion of fractions from the peak of radioactivity were analyzed on a 1 percent agarose gel. The gel was autoradiographed and cDNA fractions greater than 800 base pairs were pooled and ethanol-precipitated.

IV. Construction of the Chimera.

The cloning vector chosen in the preferred embodiment of the present invention was pBR322 (New England Nuclear). A wide variety of techniques may be employed to prepare the plasmid and gene fragment for joining, which will result in a new plasmid (or chimera) that will transform the target host, be capable of self-replication, and will utimately result in the production of the desired protein product. The technique

- 19 -

used in the preferred embodiment of the present invention was to cleave the plasmid at the Pst I site with Pst I endonuclease according to the concentration and method recommended by the manufacturer (New England Biolabs), and to prepare and join the plasmid and CDNA according to the conventional G-C tailing method as described by Maniatis, T., Fritsch, E.F. and J. Sambrook, Molecular Cloning (Cold Spring Harbor Laboratory, Cold Spring Harbor, N.Y.), 1982. The new plasmid was transformed into E. coli according to the method described by D. Hanahan, "Studies on Transformation of Escherichia coli with Plasmids", in J. Mol. Biol. 166: 557,1983.

V.   Identification of the cloned cDNA for TCGF.

Once a plasmid has been prepared and transformed into the host cell, those cells containing the plasmid with the proper cDNA insertion must be identified.  Of the various methods available, the inventors have chosen a hybridization technique as described by Hanahan, D. and M. Meselson, "Plasmid screening at high colony density", in Methods of Enzymology 100: 333, 1983.

The method involves making duplicates (on nitrocellulose filters) of established colonies which have been transformed with plasmid containing cDNA.  The colonies are probed by a radioactive oligonucleotide (5'GCAAACCATACATTCAAC 3'), chosen to hybridize to a region near the 3' end of the TCGF mRNA as sequenced by Taniguchi, et al., "Structure and expression of a cloned cDNA for human inter-leukin 2", Nature 302: 305, 1983. An autoradiogram of the second plate revealing those colonies that hybridized to the labeled oligonucleotide keyed to the original plate, permits identification and selection of colonies containing the plasmid with a high probability of also including the desired cDNA.

Approximately 15,000 cells were grown to small colonies on nitrocellulose filters (Millipore) laid on agar plates (500-1000 colonies per plate). A second nitrocellulose filter was used as a template, and the colonies were replicated and incubated until the replicated colonies were just visible. The bacteria on the replica filters were lysed, dried, prehybridized, and hybridized at 49 degrees C for 16-20 hours with $1 \times 10^6$ cpm per filter of radioactive obligonucleotide as described by Hanahan. The probe was made radioactive by labeling the hydroxyl termini of the fragments with T4-polynucleotide kinase and $(\gamma-^{32}P)$-ATP.

The filter was washed, dried in air, covered with a plastic sheet, and exposed to Kodak XAR 5 X-ray film with a DuPont Cronex intensifying screen at -80 degrees C for 1-2 days. After development to localize the colonies, a single colony was found that was markedly above background intensity. Upon careful alignment with the original bacterial colony pattern two colonies were found close to the area identified by the radioactive probe. These two colonies were grown in 2YT medium (per liter 16g Bacto

- 21 -

was isolated as described by Holmes, D.S. and M. Quigley, "A rapid boiling method for the preparation of bacterial plasmids", in Anal, Biochem. 114: 193, 1981, with some modifications. Three ml of cells were resuspended in 0.6 ml STET (8 percent sucrose, 0.5 percent Triton X-100, 50 mM EDTA pH 8.0, 10 mM Tris/HCl pH 8.0), plus 45 μl of lysozyme (Sigma Chemical Co.) at 10 mg/ml and allowed to incubate for 5 minutes at room temperature. The cells were then boiled for 1 minute and centrifuged for 10 minutes at room temperature. The cell debris was removed and pancreatic RNase (Sigma Chemical Co.) was added to 100 μg/ml and incubated for 15 minutes at 37 degrees C. In order to remove protein, the mixture was extracted with phenol:chloroform (1:1) and the aqueous layer removed. The plasmid DNA was precipitated by the addition of 30 μl of 3 M NaCl and 0.5 ml isopropanol and placed at -100 degrees C for 15 minutes. The DNA was collected by centrifugation, washed with 70 percent ethanol, dried and dissolved in 30 μl $H_2O$. When larger amounts of DNA were required, a scaled-up version of the procedure was used and the plasmid DNA banded on a CsCl gradient.

To show the identity of the cloned cDNA, it was subjected to restriction enzyme analysis and compared to the known TCGF restriction map as published by Taniguchi, et al. Further identification came from partial sequencing of the cDNA as described by Maxam, A.M. and W. Gilbert, "Sequencing end-labeled DNA with base-specific et al. Further identification came from partial sequencing of the cDNA as described by Maxam, A.M. and W. Gilbert, "Sequencing end-labeled DNA with base-specific chemical cleavages", in Methods in Enzymology 65: 499, 1980. The isolated clone, clone 1, was found to contain approximately 85 percent of the TCGF message or 690 base pairs.

In order to find a full length TCGF clone, 10,000 colonies from a second cDNA preparation were screened. The probe in this case was a restriction fragment, purified by agarose gel electrophoresis from clone 1, that was radioactively labeled by nick translation as described at Rigby, P.W.J., M. Dieckmann, C. Rhodes, and P. Berg, "Labeling deoxyribonucleic acid to high specific activity in vitro by nick translation with DNA polymerase I", in J. Mol. Biol. 113: 237, 1977.

The 10,000 colonies were replicated, lysed and dried as described. The hybridization was performed as follows: Filters were prehybridized in 6x SSC, 5X Denhardt's (SSC=0.15 M NaCl, 0.015 M Na citrate, Denhardt's= 0.02 percent Ficoll, 0.02 percent polyvinylpyrrolidone, 0.02 percent BSA) for 16-20 hours at 65 degrees C, followed by 3 hours at 65 degrees C in 6x SSC, 5x Denhardt's, 0.1 percent SDS, 10 mM EDTA, and 100 µg/ml sonicated salmon sperm DNA. Hybridization was for 16-20 hours at 65 degrees C and was done by adding $1 \times 10^6$ cpm per filter of heat denatured probe to the prehybridization.

- 23 -

A single colony was isolated and characterized by restriction map analysis and sequenced by a combination of Sanger's dideoxy method, (Sanger, F., S. Niclen and A. R. Coulson, "DNA sequencing with chain-terminating inhibitors", Proc. Natl. Acad. Sci. USA 74: 5463, 1977.), and Maxam-Gilbert chemical sequencing. The colony, identified as Escherichia coli C600/QUIDL-TF, was deposited on June 14, 1984, with the American Type Culture Collection, and is assigned ATCC Accession No. 39733.

The nucleotide sequence of the cDNA determined for the present invention differs from that published by Taniguchi, et al. at the No. 203 and 469 positions, as shown in the following sequence, corresponding to the 237 and 503 positions as given by Taniguchi, et al., the 203 position resulting in a different protein.

### TCGF cDNA SEQUENCE

```
         10        20        30        40        50
GGGGGGGGGGGGGGGGGGTACAGGATGCAACTCCTGTCTTGCATTGCACTAA

         60        70        80        90       100
GTCTTGCACTTGTCACAAACAGTGCACCTACTTCAAGTTCTACAAAGAAA

        110       120       130       140       150
ACACAGCTACAACTGGAGCATTTACTGCTGGATTTACAGATGATTTTGAA

        160       170       180       190       200
TGGAATTAATAATTACAAGAATCCCAAACTCACCAGGATGCTCACATTTA

        210       220       230       240       250
AGCTTTACATGCCCAAGAAGGCCACAGAACTGAAACATCTTCAGTGTCTA

        260       270       280       290       300
GAAGAAGAACTCAAACCTCTGGAGGAAGTGCTAAATTTAGCTCAAAGCAA

        310       320       330       340       350
AAACTTTCACTTAAGACCCAGGGACTTAATCAGCAATATCAACGTAATAG
```

```
     360      370      380      390      400
TTCTGGAACTAAAGGGATCTGAAACAACATTCATGTGTGAATATGCTGAT

       410      420      430      440      450
GAGACAGCAACCATTGTAGAATTTCTGAACAGATGGATTACCTTTTGTCA

     460      470      480      490      500
AAGCATCATCTCAACACTGACTTGATAATTAAGTGCTTCCCACTTAAAAC

     510      520      530      540      550
ATATCAGGCCTTCTATTTATTTAAATATTTAAATTTTATATTTATTGTTG

     560      570      580      590      600
AATGTATGGTTTGCTACCTATTGTAACTATTATTCTTAATCTTAAAACTA

     610      620      630      640      650
TAAATATGGATCTTTTATGATTCTTTTTGTAAGCCCTAGGGGCTCTAAAA

     660      670      680      690      700
TGGTTTCACTTATTTATCCCAAAATATTTATTATTATGTTGAATGTTAAA

     710      720      730      740      750
TATAGTATCTATGTAGATTGGTTAGTAAAACTATTTAATAAATTTGATAA

     760      770      780      790
ATATAAAAAAACACCCCCCCCCCCCCCCCCCCCCCCCCCC
```

INDUSTRIAL APPLICATION

The present invention is useful in the manufacture of T-Cell Growth Factor which has known use in the diagnosis of immune reactions and in the development of methods and materials for diagnosis and treatment of immune responses.

- 25 -

WHAT IS CLAIMED IS:

1. The bacterium Escherichia coli c600/QUIDL-TF as described and characterized herein, and as shown by examples herein.

2. A plasmid containing that portion of the following sequence of DNA which codes for T-Cell Growth Factor:

GGGGGGGGGGGGGGGGTACAGGATGCAACTCCTGTCTTGCATTGCACTAA

GTCTTGCACTTGTCACAAACAGTGCACCTACTTCAAGTTCTACAAAGAAA

ACACAGCTACAACTGGAGCATTTACTGCTGGATTTACAGATGATTTTGAA

TGGAATTAATAATTACAAGAATCCCAAACTCACCAGGATGCTCACATTTA

AGCTTTACATGCCCAAGAAGGCCACAGAACTGAAACATCTTCAGTGTCTA

GAAGAAGAACTCAAACCTCTGGAGGAAGTGCTAAATTTAGCTCAAAGCAA

AAACTTTCACTTAAGACCCAGGGACTTAATCAGCAATATCAACGTAATAG

TTCTGGAACTAAAGGGATCTGAAACAACATTCATGTGTGAATATGCTGAT

GAGACAGCAACCATTGTAGAATTTCTGAACAGATGGATTACCTTTTGTCA

AAGCATCATCTCAACACTGACTTGATAATTAAGTGCTTCCCACTTAAAAC

ATATCAGGCCTTCTATTTATTTAAATATTTAAATTTTATATTTATTGTTG

AATGTATGGTTTGCTACCTATTGTAACTATTATTCTTAATCTTAAAACTA

TAAATATGGATCTTTTATGATTCTTTTTGTAAGCCCTAGGGGCTCTAAAA

TGGTTTCACTTATTTATCCCAAAATATTTATTATTATGTTGAATGTTAAA

TATAGTATCTATGTAGATTGGTTAGTAAAACTATTTAATAAATTTGATAA

ATATAAAAAAACACCCCCCCCCCCCCCCCCCCCCCCCCCCC

3. The DNA sequence:

GTCTTGCACTTGTCACAAACAGTGCACCTACTTCAAGTTCTACAAAGAAA

ACACAGCTACAACTGGAGCATTTACTGCTGGATTTACAGATGATTTTGAA

TGGAATTAATAATTACAAGAATCCCAAACTCACCAGGATGCTCACATTTA

AGCTTTACATGCCCAAGAAGGCCACAGAACTGAAACATCTTCAGTGTCTA

GAAGAAGAACTCAAACCTCTGGAGGAAGTGCTAAATTTAGCTCAAAGCAA

AAACTTTCACTTAAGACCCAGGGACTTAATCAGCAATATCAACGTAATAG

TTCTGGAACTAAAGGGATCTGAAACAACATTCATGTGTGAATATGCTGAT

GAGACAGCAACCATTGTAGAATTTCTGAACAGATGGATTACCTTTTGTCA

AAGCATCATCTCAACACTCACTT.

4. A plasmid which includes the cDNA of Claim 3.

5. A plasmid which includes the DNA nucleotide sequence which codes for TCGF including the nucleotide sequence TTTAAGCTTTACAT.

6. <u>E. coli</u> including a plasmid as defined in Claim 5.

7. The recombinant DNA transfer vector containing nucleotide sequence TTTAAGCTTACAT and that portion of the cDNA nucleotide sequence which codes for the expression of TCGF in a microorganism.

8.   A microorganism host including the vector defined in Claim 7.

**European Patent Office**

**EUROPEAN SEARCH REPORT**

| | | DOCUMENTS CONSIDERED TO BE RELEVANT | | EP 85300902.5 |
|---|---|---|---|---|

| Category | Citation of document with indication, where appropriate, of relevant passages | Relevant to claim | CLASSIFICATION OF THE APPLICATION (Int. Cl.4) |
|---|---|---|---|
| A | EP - A1 - 0 091 539 (AJINOMOTO CO. INC. JAPANESE FOUNDATION FOR CANCER RESEARCH) <br> * Fig. 2(a); claims 19,21 * <br> -- | 1-8 | C 12 N 15/00 <br> C 12 N 1/20 <br> C 12 P 19/34 <br> //C 12 R 1:19 |
| D,A | NATURE, vol. 302, March 24, 1983, (New York, London) <br> T. TANIGUCHI et al. "Structure and expression of a cloned cDNA for human interleukin-2" <br> pages 305-310 <br> * Pages 308; fig. 3b * <br> --- - | 2-5,7 | |
| | | | **TECHNICAL FIELDS SEARCHED (Int. Cl.4)** <br><br> C 12 N <br> C 12 P |

The present search report has been drawn up for all claims

| Place of search | Date of completion of the search | Examiner |
|---|---|---|
| VIENNA | 23-10-1985 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82